# EUROPEAN PATENT SPECIFICATION

(11) **EP 2 184 063 B1**
(45) Date of publication and mention of the grant of the patent: **18.01.2017**
(21) Application number: 08783598.9
(22) Date of filing: 01.08.2008
(51) Int. Cl.: A61K 31/24, A61P 9/04

(54) **THE USE OF LEONURINE FOR THE TREATMENT OF HEART FAILURE**
VERWENDUNG VON LEONURIN ZUR BEHANDLUNG VON HERZINSUFFIZIENZ
UTILISATION DE LÉONURINE POUR LE TRAITEMENT DE L' INSUFFISANCE CARDIAQUE

(30) Priority: 02.08.2007 CN 200710044524
(43) Date of publication of application: 12.05.2010
(73) Proprietor: Fudan University, Shanghai 200433 (CN)
(72) Inventor: ZHU, Yizhun, Shanghai 200032 (CN); LIU, Xinhua, Shanghai 200032 (CN); ZHU, Yichun, Shanghai 200032 (CN); HOU, Aijun, Shanghai 200032 (CN); SUN, Xun, Shanghai 200032 (CN)
(74) Representative: Potter Clarkson LLP
(86) International application number: PCT/CN2008/001409
(87) International publication number: WO 2009/015561

(56) References cited:
- CN-A- 1 415 603
- XIONG YING ET AL: "Protective Effect of Leonurine on Acute Myocardial Ischemia Injury in Rats", ZHONGGUO SHIYAN FANGJIXUE ZAZHI = CHINESE JOURNAL OF EXPERIMENTAL TRADITIONAL MEDICAL FORMULAE,, vol. 13, no. 2, 1 February 2007 (2007-02-01), pages 21-24, XP009172766, ISSN: 1005-9903
- DATABASE WPI Week 200581 Thomson Scientific, London, GB; AN 2005-786903 XP002713577, -& CN 1 569 130 A (UNIV FUDAN) 26 January 2005 (2005-01-26)
- SUN J ET AL: "Anti-oxidative stress effects of Herba leonuri on ischemic rat hearts", LIFE SCIENCES, vol. 76, no. 26, 13 May 2005 (2005-05-13), pages 3043-3056, XP027713354, PERGAMON PRESS, OXFORD, GB ISSN: 0024-3205 [retrieved on 2005-05-13]
- CHEN C X ET AL: "Endothelium-independent vasorelaxation by leonurine, a plant alkaloid purified from Chinese motherwort.", LIFE SCIENCES, vol. 68, no. 8, 12 January 2001 (2001-01-12), pages 953-960, XP002713578, ISSN: 0024-3205
- XIONG YING ET AL: "The Equilibrium of Vasomotor Function and the Prevention of Lipid Peroxidation of Leonurine on Acute Myocardial Ischemia Injury in Rats", ZHONGGUO SHIYAN FANGJIXUE ZAZHI = CHINESE JOURNAL OF EXPERIMENTAL TRADITIONAL MEDICAL FORMULAE,, vol. 14, no. 7, 1 July 2008 (2008-07-01), pages 34-37, XP009172767, ISSN: 1005-9903
- WANG ZHAO ET AL: "Effect of leonurine on the activity of creatine kinase", JOURNAL OF ASIAN NATURAL PRODUCTS RESEARCH, vol. 6, no. 4, 1 December 2004 (2004-12-01), pages 281-287, XP009172702, GORDON AND BREACH, CHUR, CH ISSN: 1028-6020
- JIN Q.: 'Pharmacological function and application of Yimucao (Herba Leonuri) when different dosages' TIANJIN JOURNAL OF TRADIATIONAL CHINESE MEDICINE (CHINESE) vol. 20, no. 5, October 2003, pages 51 - 52
- LI S. ET AL.: 'Pharmacodynamic Research on Alkaloid and Chromocor of Leonurus Heterophyllus Sweet Isoproterenol-induced Myocardial Injury in Rats' ACTA UNIVERSITATIS TRANDITIONIS MEDICALIS SINENSIS PHARMACOLOGIAEQUE SHANGHAI (CHINESE) vol. 20, no. 1, March 2006, pages 61 - 63
- JIANG S. ET AL.: 'Effects of Alkaloid of Leonurus Heteropyllus Sweet on Heart Function After Myocardial Infarction in Rats' SH. J. TCM (CHINESE) vol. 40, no. 10, October 2006, pages 53 - 56
- CHEN S. ET AL.: 'Curative effect of herba leonuri on myocardial ischemia and its mechanism' CHINESE CRITICAL CARE MEDICINE (CHINESE) vol. 14, no. 1, January 2002, pages 19 - 22
- CHAO Z.: 'Pharmacokinetics of leonurine in rats' CLIN. PHARM. J. (CHINESE) vol. 37, no. 1, January 2002, pages 39 - 41

## Description

### FIELD OF THE INVENTION

The present invention belongs to the field of medicament technique. The present invention relates to use of Leonurine or a pharmaceutically acceptable salt thereof or a solvate thereof in the manufacture of a medicament for preventing and/or treating heart failure, and a pharmaceutical composition for preventing and/or treating heart failure.

### TECHNICAL BACKGROUND OF THE INVENTION

Chinese traditional drug, Herba Leonuri (Lamiaceae plants, ***Herba Leonuri,*** Chinese Motherwort), which has been recorded earliest in the ancient books such as "Shennong's Classic of Materia Medica", "Compendium of Materia Medica" and so on, has effects of promoting blood circulation for regulating munstrulation, and promoting diuresis to reduce edema. In "Pharmacopoeia of the People's Republic of China" (Edition 2000), it is recorded to be used for treating irregular menstruation, dysmenorrhea, amenorrhea, lochiorrhea, edema and oliguria, edema caused by acute nephritis and the like.

Leonurine is known as one of the primary active components in Chinese traditional drug Herba Leonuri. Its chemical name is 4-hydroxy-3,5-dimethoxybenzoic acid 4-guanidinobutyl ester; the molecular formula is C₁₄H₂₁N₃O₅; the structural formula is:

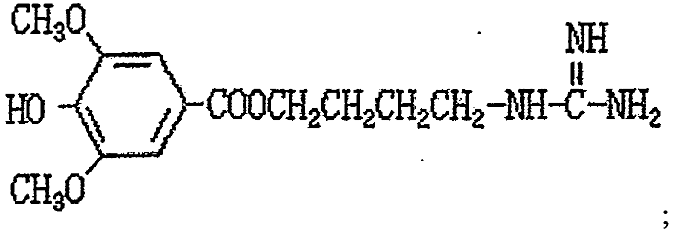

### The molecular weight: 311.33;

The physical properties: the melting point is 238°C (decomposing at this temperature), dissolving in pentanol. The melting point of Leonurine nitrite is 229-230°C, and the melting point of Leonurine hydrochloride monohydrate is 193∼194°C, dissolving in cold water, whereas only 1∼2% dissolving in hot water.

Toxicity: Leonurine is of relatively little toxicity; the rats which are celiacly injected with Leonurine 2mg per time for 4 days consecutively, show no apparent adverse reaction; and the MLD of Leonurine subcutaneously injected to frogs is 0.4∼0.6g/kg;

The component is mainly obtained from leaves of Leonurus sibiricus L.; as well as from herbs of L. heterophyllus Sweet and L. Artemisia, which are all Lamiaceae plants.

The pharmacological effects of Leonurine have been known include:
1. Effect on uterus: Leonurine has stimulative effect on the uteri of rabbits, cats, dogs, guinea pigs and many other animals. Leonurine can increase the contraction amplitude of rat's uterus isolated at pro oestrus or the isolated uterus of rat which is intramuscularly injected with 50mg of esterdiol after ovariectomization. The effects of Leonurine are in correlation with its dosage, wherein when the concentration is 0.2∼1.0mg/ml, linear relationship exists in dosage vs. tension, and the maximum tension is achieved when the concentration is above 2mg/ml. Sometimes it can be seen that Leonurine has a biphasic effect on the spontaneous contraction of the specimen. When the minimum effective amount is used or the concentration is increased suddenly (over 5 times of the initial concentration), there can be a temporary inhibition of 10-20min before inducing stimulation. High concentration (>20mg/ml) has an inhibiting effect due to its local anaesthesia to the myometrium. The contracting effect of Leonurine on uteri can last several hours, but it may be stopped by washing away the drug. 2mg/ml of Atropine cannot modify this contracting effect. Leonurine A also produces an apparent stimulation on the isolated uteri of rabbits and cats, whereas has no effect on eutopic uteri of rabbits.
2. Effect on circulatory system: Small doses of Leonurine enhance contraction of the isolated frog heart, but large doses cause inhibition contrarily which might results from the excitement of vagus nerve endings. The vascular perfusion is performed with Leonurine on frog, which shows the phenomenon of vasoconstriction which extent is proportional to the concentration of the tested solution. When an anesthetized cat is intravenously injected with Leonurine (2mg/kg), the blood pressure drops and then restores after a few minutes. Such temporary blood pressure decline still can be found after cutting the vagus nerves on both sides. If atropine is used firstly, the dropped blood pressure will be restored when Leonurine is then injected, but the effect of Leonurine is not as significant as the former one. Therefore, it can be speculated that the effect of Leonurine on lowering the blood pressure is not caused by its effect on the vagus nerve center, but may be caused by its stimulation to the vagus nerve endings. The blood vessels of the warm-blooded animals exhibit a clear expansion resulted from Leonurine which has an anti-adrenergic effect.
3. Effect on the respiratory center: Intravenous injection of Leonurine to an anesthetized cat can significantly increase the respiratory frequency and amplitude. However, when large-doses are used, its respiration transits from exciting status to suppressing status and becomes weak and irregular. After cutting the vagus nerves on both sides, Leonurine still has an exciting effect on the respiration. As a result, Leonurine is considered to maybe have a direct exciting effect on the respiratory center.
4. Other effects: Small doses of Leonurine are able to induce tensional atony of rabbit isolated intestine and enlarge the amplitude, whereas large doses of Leonurine result in a narrowed amplitude and an increased frequency. When a rabbit is intravenously injected with 1mg/kg of Leonurine, a significant increase in urine volume can be observed. Leonurine has curare-like effect on frog nerve-muscle specimens. Leonurine of high concentration can result in hemolysis of rabbit blood suspension.

It has been recorded in the documents that Leonurine is able to inhibit significantly the contraction of vascular smooth muscle by inhibiting the outside calcium ion influx and intracellular calcium ion release. However, by far, there is no report concerning the ability of Leonurine for reducing the release of lactate dehydrogenase, or preventing or treating myocardial infarction or heart failure.

### SUMMARY OF THE INVENTION

After many years of research, the present inventors have discovered and proved through lots of animal tests and clinical tests that acts as an effective active component, Leonurine not only has good effects on reducing the release of lactate dehydrogenase (LDH) and preventing or treating myocardial infarction or heart failure, but also is of quick-effectivity, little toxic side effects as well as high safety.

Several terms are defined below to facilitate the understanding of the present invention. The terms defined herein have common meanings known by a person ordinarily skilled in the related arts of the present invention.

Unless otherwise noted, as used herein, term "Leonurine" is referred to an optional racemate of Leonurine, an optional pure steromer of Leonurine, especially is an enantiomers or a diastereoisomer of Leonurine, or a mixture form of steromers of Leonurine mixed in any desired ratios; The "Leonurine" are present in their acidic forms, salt forms, or solvate forms, especially in their hydrate forms. This term further includes each enantiomers, which can be present as their salts or in free forms and are typically used with another enantiomer together which concentration is lower than 5%, preferably lower than 2%, especially lower than 1%, or are used as the mixtures of such type of enantiomers in any ratios. The mixtures of the enantiomers comprise the mixture of racemates containing substantially equal amount of two enantiomers.

Unless otherwise noted, the amount of an active component is referred to the weight of Leonurine or a pharmaceutically acceptable salt thereof or a solvate thereof.

Unless otherwise noted, as used herein, term "pharmaceutically acceptable salt" can be understood as a salt that is physiological acceptable salt, especially when applying to human beings and/or mammals as a medicament. The salt includes an acid addition salt, and the acid is, for example, hydrochloride acid, sulfuric acid, sodium bisulfate, sulphurous acid, sodium bisulfite, nitric acid, phosphoric acid, acetic acid, lactic acid, methanesulfonic acid, lactobionic acid, citric acid, oxalic acid, glutamic acid, fumaric acid, maleic acid, aspartic acid, citric acid or succinic acid. These acids mentioned above are only for illustration, but not for limitation.

Unless otherwise noted, as used herein, term "pharmaceutically acceptable carrier" is referred to the substances known in the art, which are used as the fillers or carriers in pills, tablets, capsules, and the forth. These substances are typically used as non-active components of the drugs and recognized by the health-care experts for this purpose. The compilation about pharmaceutically acceptable carriers and excipients can be found in the reference book "Handbook of Pharmaceutical excipients" (2nd Edition, edited by A. Wade and P.J. Weller, published by American Pharmaceutical Association, Washington and The Pharmaceutical Press, London, 1994), and so on.

The present invention is aimed to provide novel pharmaceutical use of Leonurine or a pharmaceutically acceptable salt thereof or a solvate thereof in the manufacture of a medicament for preventing and/or treating heart failure. The present invention is further aimed to provide a pharmaceutical composition for preventing and/or treating heart failure.

For the aims described above, the present invention provides:
The present invention provides the use of Leonurine or a pharmaceutically acceptable salt thereof or a solvate thereof in the manufacture of a medicament for preventing and/or treating heart failure.

Preferably, in the use of the present invention, the said Leonurine is a racemate of Leonurine, a pure steromer of Leonurine, especially is an enantiomer or a diastereoisomer of Leonurine, or a mixture of steromers of Leonurine mixed in any desired ratios.

Preferably, in the use of the present invention, the solvate of Leonurine is a hydrate of Leonurine.

Preferably, in the use of the present invention, the pharmaceutically acceptable salt of Leonurine is an acid addition salt of Leonurine and an acid selected from the group consisting of hydrochloride acid, sulfuric acid, sodium bisulfate, sulphurous acid, sodium bisulfites, nitric acid, phosphoric acid, acetic acid, lactic acid, methanesulfonic acid, lactobionic acid, citric acid, oxalic acid, glutamic acid, fumaric acid, maleic acid, aspartic acid, citric acid and succinic acid.

Preferably, in the use of the present invention, the dosage form of the medicament is selected from the group consisting of an oral formulation, a parenteral administration formulation, a topical administration formulation, an inhalable administration formulation and a transdermal formulation.

In a further aspect, the present invention provides a pharmaceutical composition for preventing and/or treating heart failure, which comprises Leonurine or a pharmaceutical acceptable salt thereof or a solvate thereof as an active component, and one or more pharmaceutically acceptable carriers.

Preferably, in the pharmaceutical composition of the present invention, the solvate of Leonurine is a hydrate of Leonurine.

Further preferably, in the pharmaceutical composition of the present invention, the pharmaceutically acceptable salt of the Leonurine is an acid addition salt of Leonurine and an acid selected from the group consisting of hydrochloride acid, sulfuric acid, sodium bisulfate, sulphurous acid, sodium bisulfites, nitric acid, phosphoric acid, acetic acid, lactic acid, methanesulfonic acid, lactobionic acid, citric acid, oxalic acid, glutamic acid, fumaric acid, maleic acid, aspartic acid, citric acid and succinic acid.

Preferably, in the pharmaceutical composition of the present invention, the pharmaceutical composition is an oral formulation, a parenteral administration formulation such as an injection, a topical administration formulation, an inhalable administration formulation or a transdermal formulation.

Further preferably, in the pharmaceutical composition of the present invention, the pharmaceutical composition is the oral formulation selected from the group consisting of tablets, capsules, granules, pills, drops, juices or syrups; further comprising pharmaceutical acceptable carriers selected from the group consisting of disintegrants, lubricants, binders, fillers, solvents, fragrances, sweetening agent, antioxidants, surfactants, preservatives, correctives and pigments.

Methods known in the art can be applied to prepare the composition in the present invention, and a formulation form is preferable. Such methods include a step of mixing the active component and the carriers constituting one or more auxiliary components that comprise the common components in the art such as fillers, binders, diluents, disintegrants, lubricants, colorants, flavoring agents and humectants.

The formulation suitable for the oral administration can be prepared as a dispersing unit like pills, tablets or capsules, which contains desired amount of the active component; powders or granules; solutions or suspensions. The active component can be also prepared into boluses or pastes, or can be contained in liposome.

The formulation can be prepared into a suppository or an enema for the rectal administration.

For the parenteral administration, the suitable formulation includes an aqueous and non-aqueous aseptic injection (the purity of the active component≥90%). Such formulation can be prepared into a unit-dosage form or multi-dosage form contained in a container (e.g. a sealed vial and an ampoule), can be stored under the freeze-drying (lyophilization) condition, and only need to be added an aseptic liquid (e.g. water) before using.

The formulation suitable for intranasal administration includes pressed aserosols, sprayers, or fine dusts or spray produced by an inhalant, which are calculated by the dosages.

In order to prepare such dosage unit (e.g. tablets), the application of common additives is concerned, such as fillers, colorants, polymerisable binders, and so on. Generally speaking, any pharmaceutically acceptable additives which will not interfere with the function of the active compound can be used.

The additives and/or carriers applicable in the present invention are substances known by the persons skilled in the art from the prior art, which are used to obtain herb formulations. The selection of these carriers and the amounts thereof depend on the administration patterns of the drugs, for example, oral, intravenous, intraperitoneal, intradermal, intramuscular, intranasal, buccal or topical administration. The formulations of tablets, chewable tablets, coated tablets, capsules, granules, drops, juices or syrups are suitable for the oral administration, and solutions, suspensions, dried formulations readily rehydrated and sprays are suitable for the parenteral, topical, inhalable administration. The inspection agents also might be used in rectum. Carrier membranes or patches are stored and used in the form of dissolution, optionally with other agents promoting penetrating to the skins, which are the exemplary examples suitable for the transdermal administration.

The examples of carriers and additives in the formulations for oral administration are disintegrants, lubricants, binders, fillers; alternatively are solvents, fragrances, sweetening agents; especially are carrier substances, diluents, pigments, antioxidants and so on. For the suppositories, wax and fatty acid esters can be used. For components of the parenteral administration, carrier substances, preservatives, suspending agents and the like can be used. The amount of the active component to a patient varies depending on a function relationship of the patient's weight, the administration pattern and the extent of the disease. The compound in the present invention is able to release from the formulation dosage form in a tardy manner, for the oral, rectal or transdermal administration. The continuous releasing formulations, especially the dosage forms for taken only one time each day (once per day) are suitable for indications according to the present invention.

The carriers of the dosage forms for the oral administration of the present invention can be, for example, water, ethanol, dimethyl methanol, glycerol, glycol, propylene glycol, polyglycol, polypropylene glycol, glucose, fructose, lactose, sucrose, starch, modified starch, gelatin, sorbic alcohol, inositol, mannitol, microcrystalline cellulose, methylcellulose, carboxymethyl cellulose, cellulose acetate, gum lac, cetyl alcohol, polyvinylpyrrolidone, paraffin wax, wax, natural rubber and synthetic rubber, arabic gum, alginate, dextran, saturated fatty acids and unsaturated synthetic fatty acids, stearic acid, magnesium stearate, zinc stearate, glycerin stearate, sodium lauryl sulfate, edible oil, sesame oil, coconut oil, peanut oil, soybean oil, lecithin, sodium lactate, polyoxyethylene fatty acid ester and polyoxypropylene fatty acid ester, sorbitan fatty acid ester, sorbic acid, benzoic acid, citric acid, ascorbic acid, tartaric acid, sodium chloride, potassium chloride, magnesium chloride, calcium chloride, magnesium oxide, zinc oxide, silicon dioxide, titanium oxide, titanium dioxide, magnesium sulfate, zinc sulfate, calcium sulfate, potassium carbonate, calcium phosphate, dicalcium phosphate, potassium bromide, potassium iodide, talcum, argilla, colloid, Crospovidone, agar and benlonite.

The drugs and pharmaceutical components according to the present invention can be prepared with the aid of the techniques of pharmaceutical formulations, such as the known advanced means, equipments, methods and procedures and the forth. For instance, for the solid formulation like tablets, the active component (i.e. Leonurine, pharmaceutically acceptable salts thereof or solvates thereof) can be granulated with the pharmaceutical carriers which are, for example, common components of tablets such as, corn powders, lactose, sucrose, sorbic alcohol, talcum, magnesium stearate, dicalcium phosphate or pharmaceutical acceptable colloids, to form one solid component containing homogenously distributed Leonurine or the pharmaceutical acceptable salt thereof or the solvate thereof.

The "homogenously distributed" herein can be understood as the active component (Leonurine or pharmaceutically acceptable salt thereof or the solvate thereof) is homogenously distributed in the whole component, which so that can be readily divided into unit dosage forms having the same activity, such as tablets, pills or capsules. The solid component is then divided into unit dosage forms. The tablets or pills of drugs or pharmaceutical components according to the present invention can also be coated or mixed with another component to form one sustained release dosage. The suitable coated component is a polyacid, and a mixture of the polyacid and a material such as gum lac, cetyl alcohol and/or cellulose acetate etc.

The pharmaceutical composition in the present invention can be prepared into the clinical acceptable dosage forms, such as tablets, capsules, granules, oral liquid formulation, endermic administration formulation, suppository and the like. The oral formulation like tablets, granules or capsules and the like can be prepared by adding appropriate amount of the carriers. These dosage forms can be prepared by the methods well known by a person skilled in the art. The carriers for moulding process for preparing tablets, granules, capsules and the like are the common auxiliaries, for example, starch, gelatin, arabic gum, polyglycol and the like. Additionally, the surfactants, lubricants, disintegrants, preservatives, correctives and pigments also can be used.

Vivo and/or vitro experiments of the compound Leonurine are pre-formed as follows by the present inventors, thereby fully demonstrate the applicability of the pharmaceutical use of the present invention.

The effects of Leonurine on the releasing amount of LDH into the culture media are tested by the vitro experiment to test the release of LDH.

The effects of Leonurine on the infarction area, the cardiac enzymes (creatine kinase (CK) & lactate dehydrogenase (LDH)), and the expression levels of mRNA and protein of the apoptosis genes (BAX & Bcl-2) are tested by the myocardial infarction model of ligating left anterior descending coronary artery. The testing results show that Leonurine reduces the infarction area and the level of the cardiac enzymes (LDH & CK) in plasma, increases the expression levels of mRNA and protein of Bcl-2 and decreases those of Bax.

The cardiac function and the level of Vitamin C and cysteine in plasma are tested by the heart failure model of ligating left anterior descending coronary artery. The testing results show that Leonurine is able to decrease the ventricle end-systolic pressure, increase the contraction rate, improve cardiac function, and increase the content of Vitamin C in plasma and decrease the content of cysteine in plasma.

The experimental results demonstrate that Leonurine is able to reduce the release of lactate dehydrogenase (LDH), has significant effects on preventing and/or treating acute myocardial infarction or heart failure, and can be used to prepare the medicament for preventing and treating acute myocardial infarction or heart failure.

### BRIEF DESCRIPTION OF THE DRAWINGS

The examples of the present invention below are illustrated in details which make reference to the drawings, wherein:

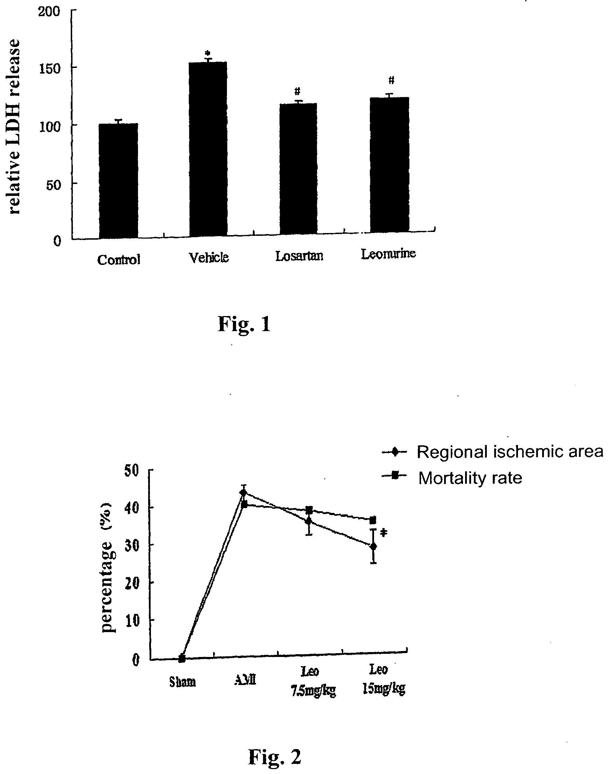

Fig. 1 is a histogram showing the relative LDH release, wherein the data are represented with the percentage of "normal group" (100%), "Control" represents the non-hypoxia group, "Vehicle" represents the hypoxia group, "Losartan" represents the group treated by losartan, and "Leonurine" represents the group treated by Leonurine; *: compared to the normal group p<0.01, #: compared to the hypoxia group p<0.01;
Fig. 2 is a graph showing Leonurine decreasing myocardial infarction area, wherein, "sham" represents the sham operation group, "AMI" represents the myocardial infarction group, "Leo7.5mg/kg" represents the group treated by Leonurine with low dosage (7.5mg/kg), "Leo15mg/kg" represents the group treated by Leonurine with high dosage (15mg/kg), *: compared to myocardial infarction group p<0.05;

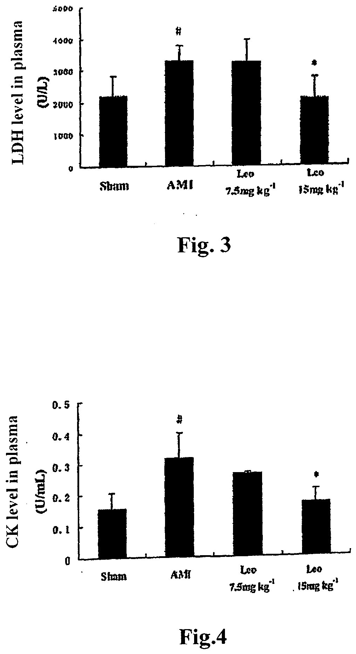

Fig. 3 is a histogram showing Leonurine reducing LDH in plasma of the rats suffered from myocardial infarction, wherein, "sham" represents the sham operation group, "AMI" represents the myocardial infarction group, "Leo7.5mg.kg⁻¹" represents the group treated by the low dosage of Leonurine (7.5mg.kg⁻¹), and "Leo15mg.kg⁻¹" represents the group treated by the high dosage of Leonurine (15mg.kg⁻¹); *: compared to the myocardial infarction group p<0.05, #: compared to the sham operation group p<0.05.
Fig. 4 is a histogram showing Leonurine reducing CK in plasma of the rats suffered from myocardial infarction, wherein, "sham" represents the sham operation group, "AMI" represents the myocardial infarction group, "Leo7.5mg.kg⁻¹" represents the group treated by the low dosage of Leonurine (7.5mg.kg⁻¹), and "Leo15mg.kg⁻¹" represents the group treated by the high dosage of Leonurine (15mg.kg⁻¹); *: compared to the myocardial infarction group p<0.05, #: compared to the sham operation group p<0.05.

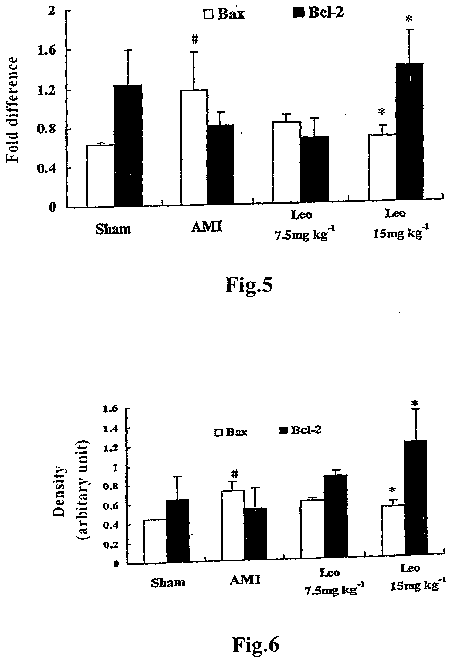

Fig. 5 is a histogram showing Leonurine reducing the expression level of mRNA of Bax and increasing that of Bcl-2, wherein, "sham" represents the sham operation group, "AMI" represents the myocardial infarction group, "Leo7.5mg.kg⁻¹" represents the group treated by the low dosage of Leonurine (7.5mg.kg⁻¹), and "Leo15mg.kg⁻¹" represents the group treated by the high dosage of Leonurine (15mg.kg⁻¹); *: compared to the myocardial infarction group p<0.05, #: compared to the sham operation group p<0.05.
Fig. 6 is a histogram showing Leonurine reducing the expression level of protein of Bax and increasing that of Bcl-2, wherein, "sham" represents the sham operation group, "AMI" represents the myocardial infarction group, "Leo 7.5mg.kg⁻¹" represents the group treated by the low dosage of Leonurine (7.5mg.kg⁻¹), and "Leo 15mg.kg⁻¹" represents the group treated by the high dosage of Leonurine (15mg.kg⁻¹); *: compared to the myocardial infarction group p<0.05, #: compared to the sham operation group p<0.05.

### PREFERRED EMBODIMENTS OF THE INVENTION

The present invention is further illustrated making reference to the specific examples. However, these examples are only used to illustrate the present invention but are not intended to limit the scope of it. Experimental methods without specific conditions in the examples below typically follow common conditions or the ones suggested by the manufacturers.

### Reference EXAMPLE 1: THE VITRO EXPERIMENT FOR TESTING THE EFFECT OF LEONURINE ON THE RELEASE OF LDH

As shown in Fig.l, losartan is used as the positive control drug in this example and the concentration of Leonurine is 10⁻⁶mol/L. The experimental data are analyzed by one-way ANOVA, p<0.01. The results show that the cell apoptosis can cause the release of LDH. By testing and analyzing the amount of LDH releasing into the culture media, the results show that Leonurine is able to reduce the release of LDH significantly, one-way ANOVA, p<0.01.

### Reference EXAMPLE 2 THE MYOCARDIAL INFARCTION MODEL TEST OF LEONURINE

As shown in Figs. 2-6, the myocardial infarction model of ligating left anterior descending coronary artery well known in the art is employed in this example, the method of the celiac injection administration is applied, the infarction area is tested by using TTC staining method, and the experimental data are analyzed by one-way ANOVA, which show that Leonurine is able to decrease the myocardial infarction area significantly p<0.05.

The myocardial infarction can result in the cardiac enzymes (LDH & CK) releasing into plasma, and Leonurine is able to reduce the LDH and CK level in plasma significantly and decrease the extent of myocardial damages, one-way ANOVA, p<0.05.

Bax is a proapoptosis gene, and Bcl-2 is an antiapoptosis gene. Leonurine is able to reduce the expression levels of mRNA and protein of Bax significantly, and increase the expression levels of mRNA and protein of Bcl-2 remarkably.

The experimental results show that Leonurine has a significant effect on improving and treating acute myocardial infarction, which can be used to prepare the medicament for preventing and treating acute myocardial infarction.

### EXAMPLE 3: THE HEART FAILURE MODEL TEST OF LEONURINE

The heart failure model of ligating left anterior descending coronary artery well known in the art is employed in this example, and the method of the celiac gastric perfusion administration is applied. The method of artery and ventricle cannulation is used to test the cardiac function. The result shows that Leonurine is able to reduce the ventricle end-systolic pressure, increase the contraction rate, and improve cardiac function. One-way ANOVA, p<0.05.

The contents of Vitamin C and cysteine in plasma are detected with Capillary Electrophoresis. The results show that Leonurine is able to increase the content of Vitamin C in plasma and decrease the content of cysteine in plasma, one-way ANOVA, p<0.05.

The cardiac function testing results of heart failure model are given in Table 1, which show that Leonurine enhances the cardiac function of the rats suffered from heart failure, wherein, *: compared to heart failure group p<0.05, #: compared to sham operation group p<0.05.

**Table 1 Testing results of the cardiac function of the heart failure model**

| **parameters** | **Sham (n=6)** | **Group of heart failure model (HF) + physiological saline solution (n=8)** | **Group of heart failure model (HF) + Leonurine** | |
|---|---|---|---|---|
| | | | **15mg/kg (n=8)** | **30mg/kg (n=8)** |
| **HR, times/min** | 410±22 | 408±78 | 441±29 | 427±78 |
| **LVSP, mmHg** | 186.73±36.71 | 113.15±12.9^{#} | 143.77±26.82 | 126.37±2.28 |
| **LVEDP,mmHg** | 3.88±1.54 | 15.54±1.36^{#} | 11.53±1.83* | 13±5.96 |
| **dP/dt, mmHg/s** | 6139.47±635.55 | 3047.26±322.71^{#} | 4205±412.71* | 4095±433.45* |
| **-dP/dt, mmHg/s** | -5571.52±626.56 | -2599.37±587.44^{#} | -4368.83±662.12* | -3276.62±283.77 |

The experimental results are given in Table 2 below, which show that Leonurine increases the content of Vitamin C in plasma and decreases the content of cysteine in plasma, wherein, *: compared to heart failure group p<0.05, **: compared to heart failure group p<0.01, #: compared to sham operation group p<0.05.

**Table 2 Leonurine increasing the content of Vitamin C in plasma and decreasing the content of cysteine in plasma**

| **compounds** | **Sham (n=4)** | **Group of heart failure model(HF)+ physiological saline solution (n=4)** | **Leonurine Group** | |
|---|---|---|---|---|
| | | | **15mg/kg (n=4)** | **30 mg/kg (n=4)** |
| **Leonurine,µmol/l** | 0 | 0 | 7.42±3.13* | 48.04±10.95** |
| **cysteine, µml/l** | 29.8 ±2.29 | 55±14.31# | 31.3±21.32* | 35.29±9.73 |
| **ascorbic acid(Vc), µmol/l** | 11.63±1.66 | 8.42±2.93 | 26.02±3.95** | 39.79±4.33** |

## Claims

1. Use of Leonurine or a pharmaceutically acceptable salt thereof or a solvate thereof in the manufacture of a medicament for preventing and/or treating heart failure.

2. The use of Claim 1, wherein the Leonurine is a racemate of Leonurine, or a pure steromer of Leonurine, preferably wherein Leonurine is an enantiomer or a diastereoisomer of Leonurine, or a mixture of the steromers of Leonurine mixed in any desired ratio.

3. The use of Claim 1 or 2, wherein the solvate of Leonurine is a hydrate of Leonurine.

4. The use of Claim 1 or 2, wherein the pharmaceutically acceptable salt of Leonurine is an acid addition salt of Leonurine and an acid selected from the group consisting of hydrochloride acid, sulfuric acid, sodium bisulfate, sulphurous acid, sodium bisulfite, nitric acid, phosphoric acid, acetic acid, lactic acid, methanesulfonic acid, lactobionic acid, citric acid, oxalic acid, glutamic acid, fumaric acid, maleic acid, aspartic acid, citric acid and succinic acid.

5. The use of any one of Claims 1 to 4, wherein the dosage form of the medicament is selected from the group consisting of an oral formulation, a parenteral administration formulation, a topical administration formulation, an inhalable administration formulation and a transdermal formulation.

6. Leonurine or a pharmaceutically acceptable salt thereof or a solvate thereof for use in the prevention and/or treatment of heart failure.

7. The compound for use of Claim 6, wherein the Leonurine is a racemate of Leonurine, or a pure steromer of Leonurine, preferably wherein Leonurine is an enantiomer or a diastereoisomer of Leonurine, or a mixture of the steromers of Leonurine mixed in any desired ratio.

8. The compound for use of Claim 6 or 7, wherein the solvate of Leonurine is a hydrate of Leonurine.

9. The compound for use of Claim 6 or 7, wherein the pharmaceutically acceptable salt of Leonurine is an acid addition salt of Leonurine and an acid selected from the group consisting of hydrochloride acid, sulfuric acid, sodium bisulfate, sulphurous acid, sodium bisulfite, nitric acid, phosphoric acid, acetic acid, lactic acid, methanesulfonic acid, lactobionic acid, citric acid, oxalic acid, glutamic acid, fumaric acid, maleic acid, aspartic acid, citric acid and succinic acid.

10. The compound for use of any one of Claims 6 to 9, wherein the dosage form of the medicament is selected from the group consisting of an oral formulation, a parenteral administration formulation, a topical administration formulation, an inhalable administration formulation and a transdermal formulation.

11. A pharmaceutical composition for use in preventing and/or treating heart failure, comprising:
Leonurine or a pharmaceutically acceptable salt thereof or a solvate thereof as an active component, and one or more pharmaceutically acceptable carriers.

12. The pharmaceutical composition for use in preventing and/or treating heart failure according to Claim 11, wherein the solvate of Leonurine is a hydrate of Leonurine.

13. The pharmaceutical composition for use in preventing and/or treating heart failure according to Claim 11, wherein the pharmaceutically acceptable salt of Leonurine is an acid addition salt of Leonurine and an acid selected from the group consisting of hydrochloride acid, sulfuric acid, sodium bisulfate, sulphurous acid, sodium bisulfite, nitric acid, phosphoric acid, acetic acid, lactic acid, methanesulfonic acid, lactobionic acid, citric acid oxalic acid, glutamic acid, fumaric acid, maleic acid, aspartic acid, citric acid and succinic acid.

14. The pharmaceutical composition for use in preventing and/or treating heart failure according to any one of Claims 11 to 13, wherein the pharmaceutical composition is an oral formulation, a parenteral administration formulation such as an injection, a topical administration formulation, an inhalable administration formulation or a transdermal formulation.

15. The pharmaceutical composition for use in preventing and/or treating heart failure according to Claim 14, wherein the pharmaceutical composition is an oral formulation selected from the group consisting of tablets, capsules, granules, pills, drops, juices or syrups; further comprising pharmaceutical acceptable carriers selected from the group consisting of disintegrants, lubricants, binders, fillers, solvents, fragrances, sweetening agent, antioxidants, surfactants, preservatives, correctives and pigments.

## Patentansprüche

1. Gebrauch von Leonurin oder einem pharmazeutisch unbedenklichen Salz davon oder einem Solvat davon beim Herstellen eines Medikaments zum Vorbeugen und/oder Behandeln von Herzinsuffizienz.

2. Gebrauch nach Anspruch 1, wobei das Leonurin ein Racemat von Leonurin oder ein reines Stereomer von Leonurin ist, vorzugsweise wobei Leonurin ein Enantiomer oder ein Diastereoisomer von Leonurin oder eine in einem beliebigen Verhältnis gemischte Mischung der Stereomere von Leonurin ist.

3. Gebrauch nach Anspruch 1 oder 2, wobei das Solvat von Leonurin ein Hydrat von Leonurin ist.

4. Gebrauch nach Anspruch 1 oder 2, wobei das pharmazeutisch unbedenkliche Salz von Leonurin ein Säureadditionssalz von Leonurin und einer Säure ist, ausgewählt aus der Gruppe bestehend aus Hydrochloridsäure, Schwefelsäure, Natriumbisulfat, schwefliger Säure, Natriumbisulfit, Salpetersäure, Phosphorsäure, Essigsäure, Milchsäure, Methansulfonsäure, Lactobionsäure, Citronensäure, Oxalsäure, Glutaminsäure, Fumarsäure, Maleinsäure, Asparaginsäure, Citronensäure und Bernsteinsäure.

5. Gebrauch nach einem der Ansprüche 1 bis 4, wobei die Dosierungsform des Medikaments ausgewählt ist aus der Gruppe bestehend aus einer oralen Formulierung, einer Formulierung zur parenteralen Verabreichung, einer Formulierung zur topischen Verabreichung, einer Formulierung zur inhalierbaren Verabreichung und einer transdermalen Formulierung.

6. Leonurin oder pharmazeutisch unbedenkliches Salz davon oder Solvat davon zum Gebrauch beim Vorbeugen und/oder Behandeln von Herzinsuffizienz.

7. Verbindung zum Gebrauch nach Anspruch 6, wobei das Leonurin ein Racemat von Leonurin oder ein reines Stereomer von Leonurin ist, vorzugsweise wobei Leonurin ein Enantiomer oder ein Diastereoisomer von Leonurin oder eine in einem beliebigen Verhältnis gemischte Mischung der Stereomere von Leonurin ist.

8. Verbindung zum Gebrauch nach Anspruch 6 oder 7, wobei das Solvat von Leonurin ein Hydrat von Leonurin ist.

9. Verbindung zum Gebrauch nach Anspruch 6 oder 7, wobei das pharmazeutisch unbedenkliche Salz von Leonurin ein Säureadditionssalz von Leonurin und einer Säure ist, ausgewählt aus der Gruppe bestehend aus Hydrochloridsäure, Schwefelsäure, Natriumbisulfat, schwefliger Säure, Natriumbisulfit, Salpetersäure, Phosphorsäure, Essigsäure, Milchsäure, Methansulfonsäure, Lactobionsäure, Citronensäure, Oxalsäure, Glutaminsäure, Fumarsäure, Maleinsäure, Asparaginsäure, Citronensäure und Bernsteinsäure.

10. Verbindung zum Gebrauch nach einem der Ansprüche 6 bis 9, wobei die Dosierungsform des Medikaments ausgewählt ist aus der Gruppe bestehend aus einer oralen Formulierung, einer Formulierung zur parenteralen Verabreichung, eine Formulierung zur topischen Verabreichung, eine Formulierung zur inhalierbaren Verabreichung und eine transdermale Formulierung.

11. Pharmazeutische Zusammensetzung zum Gebrauch beim Vorbeugen und/oder Behandeln von Herzinsuffizienz, Folgendes umfassend:
Leonurin oder ein pharmazeutisch unbedenkliches Salz davon oder ein Solvat davon als ein Wirkstoff sowie einen oder mehrere pharmazeutisch unbedenkliche Trägerstoffe.

12. Pharmazeutische Zusammensetzung zum Gebrauch beim Vorbeugen und/oder Behandeln von Herzinsuffizienz nach Anspruch 11, wobei das Solvat von Leonurin ein Hydrat von Leonurin ist.

13. Pharmazeutische Zusammensetzung zum Gebrauch beim Vorbeugen und/oder Behandeln von Herzinsuffizienz nach Anspruch 11, wobei das pharmazeutisch unbedenkliche Salz von Leonurin ein Säureadditionssalz von Leonurin und einer Säure ist, ausgewählt aus der Gruppe bestehend aus Hydrochloridsäure, Schwefelsäure, Natriumbisulfat, schwefliger Säure, Natriumbisulfit, Salpetersäure, Phosphorsäure, Essigsäure, Milchsäure, Methansulfonsäure, Lactobionsäure, Citronensäure, Oxalsäure, Glutaminsäure, Fumarsäure, Maleinsäure, Asparaginsäure, Citronensäure und Bernsteinsäure.

14. Pharmazeutische Zusammensetzung zum Gebrauch beim Vorbeugen und/oder Behandeln von Herzinsuffizienz nach einem der Ansprüche 11 bis 13, wobei die pharmazeutische Zusammensetzung eine orale Formulierung, eine Formulierung zur parenteralen Verabreichung wie etwa eine Injektion, eine Formulierung zur topischen Verabreichung, eine Formulierung zur inhalierbaren Verabreichung oder eine transdermale Formulierung ist.

15. Pharmazeutische Zusammensetzung zum Gebrauch beim Vorbeugen und/oder Behandeln von Herzinsuffizienz nach Anspruch 14, wobei die pharmazeutische Zusammensetzung eine orale Formulierung ist, ausgewählt aus der Gruppe bestehend aus Tabletten, Kapseln, Granulatkörnchen, Pillen, Tropfen, Säften oder Sirupen; ferner umfassend pharmazeutisch unbedenkliche Trägerstoffe, ausgewählt aus der Gruppe bestehend aus Sprengmitteln, Gleitmitteln, Bindemitteln, Füllstoffen, Lösungsmitteln, Duftstoffen, Süßstoff, Antioxidationsmitteln, Tensiden, Konservierungsmitteln, Abhilfen und Pigmenten.

## Revendications

1. Utilisation de léonurine ou d'un sel pharmaceutiquement acceptable de celle-ci ou d'un solvate de celle-ci dans la fabrication d'un médicament pour la prévention et/ou le traitement de l'insuffisance cardiaque.

2. Utilisation selon la revendication 1, dans laquelle la léonurine est un racémate de léonurine ou un stéréomère pur de léonurine, de préférence dans laquelle la léonurine est un énantiomère ou un diastéréoisomère de léonurine, ou un mélange des stéréomères de léonurine mélangés dans un quelconque rapport souhaité.

3. Utilisation selon la revendication 1 ou 2, dans laquelle le solvate de léonurine est un hydrate de léonurine.

4. Utilisation selon la revendication 1 ou 2, dans laquelle le sel pharmaceutiquement acceptable de léonurine est un sel d'addition acide de léonurine et un acide choisi dans le groupe constitué par l'acide chlorhydrique, l'acide sulfurique, le bisulfate de sodium, l'acide sulfureux, le bisulfite de sodium, l'acide nitrique, l'acide phosphorique, l'acide acétique, l'acide lactique, l'acide méthanesulfonique, l'acide lactobionique, l'acide citrique, l'acide oxalique, l'acide glutamique, acide fumarique, l'acide maléique, l'acide aspartique, l'acide citrique et l'acide succinique.

5. Utilisation selon l'une quelconque des revendications 1 à 4, dans laquelle la forme pharmaceutique du médicament est choisie dans le groupe constitué par une formulation administrable par voie orale, une formulation administrable par voie parentérale, une formulation administrable par voie topique, une formulation administrable par inhalation et une formulation administrable par voie transdermique.

6. Léonurine ou sel pharmaceutiquement acceptable de celle-ci ou solvate de celle-ci pour une utilisation dans la prévention et/ou le traitement de l'insuffisance cardiaque.

7. Composé pour une utilisation selon la revendication 6, dans lequel la léonurine est un racémate de léonurine ou un stéréomère pur de léonurine, de préférence dans lequel la léonurine est un énantiomère ou un diastéréoisomère de léonurine, ou un mélange des stéréomères de léonurine mélangés dans un quelconque rapport souhaité.

8. Composé pour une utilisation selon la revendication 6 ou 7, dans lequel le solvate de léonurine est un hydrate de léonurine.

9. Composé pour une utilisation selon la revendication 6 ou 7, dans lequel le sel pharmaceutiquement acceptable de léonurine est un sel d'addition acide de léonurine et un acide choisi dans le groupe constitué par l'acide chlorhydrique, l'acide sulfurique, le bisulfate de sodium, l'acide sulfureux, le bisulfite de sodium, l'acide nitrique, l'acide phosphorique, l'acide acétique, l'acide lactique, l'acide méthanesulfonique, l'acide lactobionique, l'acide citrique, l'acide oxalique, l'acide glutamique, l'acide fumarique, l'acide maléique, l'acide aspartique, l'acide citrique et l'acide succinique.

10. Composé pour une utilisation selon l'une quelconque des revendications 6 à 9, dans lequel la forme pharmaceutique du médicament est choisie dans le groupe constitué par une formulation administrable par voie orale, une formulation administrable par voie parentérale, une formulation administrable par voie topique, une formulation administrable par inhalation et une formulation administrable par voie transdermique.

11. Composition pharmaceutique pour une utilisation dans la prévention et/ou le traitement de l'insuffisance cardiaque, comprenant :
de la léonurine ou un sel pharmaceutiquement acceptable de celle-ci ou un solvate de celle-ci en tant que principe actif, et un ou plusieurs vecteurs pharmaceutiquement acceptables.

12. Composition pharmaceutique pour une utilisation dans la prévention et/ou le traitement de l'insuffisance cardiaque selon la revendication 11, dans laquelle le solvate de léonurine est un hydrate de léonurine.

13. Composition pharmaceutique pour une utilisation dans la prévention et/ou le traitement de l'insuffisance cardiaque selon la revendication 11, dans lequel le sel pharmaceutiquement acceptable de léonurine est un sel d'addition acide de léonurine et un acide choisi dans le groupe constitué par l'acide chlorhydrique, l'acide sulfurique, le bisulfate de sodium, l'acide sulfureux, le bisulfite de sodium, l'acide nitrique, l'acide phosphorique, l'acide acétique, l'acide lactique, l'acide méthanesulfonique, l'acide lactobionique, l'acide citrique, l'acide oxalique, l'acide glutamique, l'acide fumarique, l'acide maléique, l'acide aspartique, l'acide citrique et l'acide succinique.

14. Composition pharmaceutique pour une utilisation dans la prévention et/ou le traitement de l'insuffisance cardiaque selon l'une quelconque des revendications 11 à 13, laquelle composition pharmaceutique est une formulation administrable par voie orale, une formulation administrable par voie parentérale telle qu'une injection, une formulation administrable par voie topique, une formulation administrable par inhalation ou une formulation administrable par voie transdermique.

15. Composition pharmaceutique pour une utilisation dans la prévention et/ou le traitement de l'insuffisance cardiaque selon la revendication 14, laquelle composition pharmaceutique est une formulation administrable par voie orale choisie dans le groupe constitué par les comprimés, les capsules, les granules, les pilules, les gouttes, les jus ou les sirops ; comprenant en outre les vecteurs pharmaceutiquement acceptables choisis dans le groupe constitué par les délitants, les lubrifiants, les liants, les agents de remplissage, les solvants, les arômes, les édulcorants, les antioxydants, les tensioactifs, les conservateurs, les correcteurs et les pigments.
